(19) 

Europäisches
Patentamt
European
Patent Office
Office européen
des brevets

(11) **EP 4 432 296 A1**

(12) **EUROPEAN PATENT APPLICATION**
published in accordance with Art. 153(4) EPC

(43) Date of publication:
**18.09.2024 Bulletin 2024/38**

(21) Application number: **22891977.5**

(22) Date of filing: **08.11.2022**

(51) International Patent Classification (IPC):
**G16H 20/30** (2018.01)

(86) International application number:
**PCT/CN2022/130641**

(87) International publication number:
**WO 2023/083183 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.11.2021 CN 202111336086**

(71) Applicants:
 • **BOE Technology Group Co., Ltd.**
 **Beijing 100015 (CN)**
 • **Beijing BOE Technology Development Co., Ltd.**
 **Beijing 100176 (CN)**

(72) Inventors:
 • **SU, Jing**
 **Beijing 100176 (CN)**
 • **ZHAO, Junjie**
 **Beijing 100176 (CN)**
 • **CHEN, Shaobei**
 **Beijing 100176 (CN)**
 • **CHU, Xiao**
 **Beijing 100176 (CN)**

(74) Representative: **Santarelli**
 **Tour Trinity**
 **1 bis Esplanade de la Défense**
 **92035 Paris La Défense Cedex (FR)**

(54) **FITNESS PROGRAM INFORMATION RECOMMENDATION METHOD AND DEVICE**

(57)   The present disclosure relates to the technical field of computers, and relates to a fitness program information recommendation method and device. The recommendation method comprises: according to physiological feature information of a user requiring recommendation, determining a matching human body model of the physiological feature information among multiple analogous human body models, the multiple analogous human body models being generated according to physiological feature information of multiple analogous users; and according to fitness program information of the analogous user associated with the matching human body model, determining fitness program information for the user requiring recommendation. The technical solution of the present disclosure can recommend fitness program information to a user in a more targeted manner, thereby solving the technical problem of poor user experience caused by inaccurate fitness program information.

FIG. 1

EP 4 432 296 A1

**EP 4 432 296 A1**

## Description

### CROSS-REFERENCE TO RELATED APPLICATIONS

The present disclosure is based on and claims priority of Chinese application for invention No. 202111336086.1, filed on Nov. 12, 2021, the disclosure of which is hereby incorporated into this disclosure by reference in its entirety.

### TECHNICAL FIELD

This disclosure relates to the field of computer technology, in particular to a recommendation method for fitness regimen information, a recommendation apparatus for fitness regimen information and a non-transitory computer-readable storage medium.

### BACKGROUND

In related technologies, application software determines a user's current body shape and an expected body shape from some fixed types of body shape to recommend a fitness regimen.

### SUMMARY

According to some embodiments of the present disclosure, there is provided a recommendation method for fitness regimen information, comprising: determining, based on physiological feature information of a user to be recommended, a matching human body model for the physiological feature information from a plurality of comparable human body models, the plurality of comparable human body models being generated based on physiological feature information of a plurality of comparable users; determining fitness regimen information for the user to be recommended based on fitness regimen information of a comparable user associated with the matching human body model.

In some embodiments, determining fitness regimen information for the user to be recommended based on fitness regimen information of a comparable user associated with the matching human body model comprises: displaying fitness effect information of the comparable user associated with the matching human body model and corresponding fitness regimen information of the fitness effect information to the user to be recommended; determining the fitness regimen information of the user to be recommended based on a selection of the fitness effect information and the corresponding fitness regimen information by the user to be recommended.

In some embodiments, determining fitness regimen information for the user to be recommended based on fitness regimen information of a comparable user associated with the matching human body model comprises: according to a target part selected by the user to be recommended, displaying a plurality of fitness effect information of the target part associated with the matching human body model to the user to be recommended, training periods and training intensities corresponding to the plurality of fitness effect information; determining, based on target fitness effect information selected from the plurality of fitness effect information by the user to be recommended, a training period and a training intensity corresponding to the target fitness effect information as the fitness regimen information.

In some embodiments, determining, based on physiological feature information of a user to be recommended, a matching human body model for the physiological feature information from a plurality of comparable human body models comprises: generating a user human body model of the user to be recommended based on the physiological feature information of the user to be recommended; determining the matching human body model based on a degree of similarity between the user human body model and the plurality of comparable human body models.

In some embodiments, determining the matching human body model based on a degree of similarity between the user human body model and the plurality of comparable human body models comprises: determining a comparable human body model with an overlap area greater than a threshold value with the user human body model as the matching human body model.

In some embodiments, determining the matching human body model based on a degree of similarity between the user human body model and the plurality of comparable human body models comprises: scaling the user human body model proportionally to generate a scaled user human body model which matches the size of the plurality of comparable human body models; determining the matching human body model based on a degree of similarity between the scaled user human body model and the plurality of comparable human body models.

In some embodiments, generating a user human body model of the user to be recommended based on physiological feature information of the user to be recommended comprises: generating a user current human body model of the user to be recommended based on the physiological feature information of the user to be recommended; generating a user target human body model according to an adjustment of the user to be recommended for the user current human body model based on a fitness target; determining the matching human body model based on a degree of similarity

2

between the user human body model and the plurality of comparable human body models comprises: determining the matching human body model based on a degree of similarity between the user target human body model and the plurality of comparable human body models.

In some embodiments, determining, based on physiological feature information of a user to be recommended, a matching human body model for the physiological feature information from a plurality of comparable human body models comprises: determining the matching human body model from the plurality of comparable human body models based on the physiological feature information and living habit information of the user to be recommended.

In some embodiments, determining the matching human body model from the plurality of comparable human body models based on the physiological feature information and living habit information of the user to be recommended comprises: determining a plurality of candidate human body models from the plurality of comparable human body models based on the physiological feature information of the user to be recommended; determining the matching human body model from the plurality of candidate human body models based on the living habit information of the user to be recommended; or determining a plurality of candidate human body models from the plurality of comparable human body models based on the living habit information of the user to be recommended; determining the matching human body model from the plurality of candidate human body models based on the physiological feature information of the user to be recommended.

In some embodiments, determining the matching human body model from the plurality of comparable human body models based on the physiological feature information and living habit information of the user to be recommended comprises: determining the matching human body model from the plurality of comparable human body models, also based on health condition information of the user to be recommended.

In some embodiments, determining the matching human body model from the plurality of comparable human body models also based on health condition information of the user to be recommended comprises: determining a plurality of candidate human body models from the plurality of comparable human body models based on the physiological feature information of the user to be recommended; determining the matching human body model from the plurality of candidate human body models based on the living habit information and the health condition information of the user to be recommended; or determining a plurality of candidate human body models from the plurality of comparable human body models based on the living habit information and the health condition information of the user to be recommended; determining the matching human body model from the plurality of candidate human body models based on the physiological feature information of the user to be recommended.

In some embodiments, the recommendation method further comprises: obtaining physiological feature information after training of the user to be recommended after training for a preset period of time based on the fitness regimen information; generating a human body model after training based on the physiological feature information after training; determining a matching human body model for the human body model after training from the plurality of comparable human body models based on the human body model after training; determining new fitness regimen information for the user to be recommended based on fitness regimen information associated with the matching human body model for the human body model after training.

In some embodiments, determining, based on physiological feature information of a user to be recommended, a matching human body model for the physiological feature information from a plurality of comparable human body models comprises: generating a user feature vector based on the physiological feature information of the user to be recommended; determining a matching user from a plurality of comparable users based on a degree of similarity between the user feature vector and comparable feature vectors of the plurality of comparable users, the comparable feature vectors being generated based on physiological feature information of the comparable users; determining the comparable human body model of the matching user as the matching human body model.

In some embodiments, generating a user feature vector based on the physiological feature information of the user to be recommended comprises: generating the user feature vector based on at least one of living habit information of the user to be recommended and a weight of the living habit information, health condition information of the user to be recommended and a weight of the health condition information, as well as the physiological feature information of the user to be recommended and a weight of the physiological feature information, the comparable feature vector of the comparable user being generated based on at least one of living habit information of the comparable user and a weight of the living habit information, health condition information of the comparable user and a weight of the health condition information, as well as physiological feature information of the comparable user and a weight of the physiological feature information.

In some embodiments, the weight of the health condition information of the user to be recommended is greater than the weight of the living habit information of the user to be recommended, and the weight of the living habit information of the user to be recommended is greater than the weight of the physiological feature information of the user to be recommended; the weight of the health condition information of the comparable user is greater than the weight of the living habit information of the comparable user, and the weight of living habit information of the comparable user is greater than the weight of the physiological feature information of the comparable user.

In some embodiments, determining a matching user from a plurality of comparable users based on a degree of similarity between the user feature vector and comparable feature vectors of the plurality of comparable users comprises: determining, using a machine learning model, a user training target vector of the user to be recommended based on the user feature vector, the user training target vector comprising training target information corresponding to a body part which the user to be recommended wants to achieve; determining a user feature matrix of the user to be recommended based on the user feature vector and the user training target vector; determining a matching user from a plurality of comparable users based on a degree of similarity between user feature matrix and comparable feature matrices of the plurality of comparable users, the comparable feature matrices being generated based on the comparable feature vectors and comparable training target vectors of the plurality of comparable users.

In some embodiments, the machine learning model is trained using the comparable feature vectors as inputs and the comparable training target vectors as outputs.

In some embodiments, the recommendation method further comprises: determining a preference matrix based on degrees of preference of a comparable user for different fitness effect information; determining a match matrix based on degrees of match between fitness effect information and different fitness regimen information; determining fitness regimen information for the user to be recommended based on fitness regimen information of a comparable user associated with the matching human body model comprises: determining degrees of recommendation of the comparable user for different fitness regimen information based on the preference matrix and the match matrix; determining the fitness regimen information of the user to be recommended based on a degree of recommendation of a comparable user associated with the matching human body model selected by the user to be recommended.

According to some other embodiments of the present disclosure, there is provided A recommendation apparatus for fitness regimen information, comprising: a match unit for, based on physiological feature information of a user to be recommended, determining a matching human body model for the physiological feature information from a plurality of comparable human body models, the plurality of comparable human body models being generated based on physiological feature information of a plurality of comparable users; a recommendation unit for determining fitness regimen information for the user to be recommended based on fitness regimen information of a comparable user associated with the matching human body model.

In some embodiments, the recommendation unit displays fitness effect information of the comparable user associated with the matching human body model and corresponding fitness regimen information of the fitness effect information to the user to be recommended; determines fitness regimen information of the user to be recommended based on a selection of the fitness effect information and the corresponding fitness regimen information by the user to be recommended.

In some embodiments, the recommendation unit displays, according to a target part selected by the user to be recommended, a plurality of fitness effect information of the target part associated with the matching human body model, training periods and training intensities corresponding to the plurality of fitness effect information, to the user to be recommended; determines, based on target fitness effect information selected from the plurality of fitness effect information by the user to be recommended, a training period and a training intensity corresponding to the target fitness effect information as the fitness regimen information.

In some embodiments, the match unit generates a user human body model of the user to be recommended based on the physiological feature information of the user to be recommended; determines a matching human body model based on a degree of similarity between the user human body model and a plurality of comparable human body models.

In some embodiments, the match unit determines a comparable human body model with an overlap area greater than a threshold value with the user human body model as a matching human body model.

In some embodiments, the match unit proportionally scales the user human body model to generate a scaled user human body model which matches the size of the comparable human body models; determines a matching human body model based on a degree of similarity between the scaled user human body model and the comparable human body models.

In some embodiments, the match unit generates a user current human body model of the user to be recommended based on the physiological feature information of the user to be recommended; generates a user target human body model according to an adjustment of the user to be recommended for the user current human body model based on a fitness target; the match unit determines the matching human body model based on a degree of similarity between the user target human body model and the plurality of comparable human body models.

In some embodiments, the match unit determines the matching human body model from the plurality of comparable human body models based on the physiological feature information and living habit information of the user to be recommended.

In some embodiments, the match unit determines a plurality of candidate human body models from a plurality of comparable human body models based on the physiological feature information of the user to be recommended; determines the matching human body model from the plurality of candidate human body models based on the living habit information of the user to be recommended; or determines a plurality of candidate human body models from the plurality of comparable human body models based on the living habit information of the user to be recommended; determines the matching human body model from the plurality of candidate human body models based on the physiological feature information of the user to be recommended.

In some embodiments, the match unit determines a matching human body model from the plurality of comparable human body models, also based on health condition information of the user to be recommended.

In some embodiments, the match unit determines a plurality of candidate human body models from a plurality of comparable human body models based on the physiological feature information of the user to be recommended; determines the matching human body model from the plurality of candidate human body models based on the living habit information and the health condition information of the user to be recommended; or determines a plurality of candidate human body models from the plurality of comparable human body models based on the living habit information and the health condition information of the user to be recommended; determines the matching human body model from the plurality of candidate human body models based on the physiological feature information of the user to be recommended.

In some embodiments, the match unit acquires physiological feature information after training of the user to be recommended after training for a preset period of time based on the fitness regimen information; generates a human body model after training based on the physiological feature information after training; determines a matching human body model for the human body model after training from the plurality of comparable human body models based on the human body model after training; the recommendation unit determines new fitness regimen information for the user to be recommended based on fitness regimen information associated with the matching human body model for the human body model after training.

In some embodiments, the match unit generates a user feature vector based on the physiological feature information of the user to be recommended; determines a matching user from a plurality of comparable users based on a degree of similarity between the user feature vector and comparable feature vectors of the plurality of comparable users, the comparable feature vectors being generated based on physiological feature information of the comparable users; determines the comparable human body model of the matching user as the matching human body model.

In some embodiments, the match unit generates a user feature vector based on at least one of living habit information of the user to be recommended and a weight of the living habit information, health condition information of the user to be recommended and a weight of the health condition information, as well as the physiological feature information of the user to be recommended and a weight of the physiological feature information; generates a comparable feature vector based on at least one of living habit information of the comparable user and a weight of the living habit information, health condition information of the comparable user and a weight of the health condition information, as well as physiological feature information of the comparable user and a weight of the physiological feature information.

In some embodiments, the weight of the health condition information of the user to be recommended is greater than the weight of the living habit information of the user to be recommended, and the weight of the living habit information of the user to be recommended is greater than the weight of the physiological feature information of the user to be recommended; the weight of the health condition information of the comparable user is greater than the weight of the living habit information of the comparable user, and the weight of living habit information of the comparable user is greater than the weight of the physiological feature information of the comparable user.

In some embodiments, the match unit determines, using a machine learning model, a user training target vector of the user to be recommended based on the user feature vector, the user training target vector comprising training target information corresponding to a body part which the user to be recommended wants to achieve; determines a user feature matrix of the user to be recommended based on the user feature vector and the user training target vector; determines a matching user from a plurality of comparable users based on a degree of similarity between user feature matrix and comparable feature matrices of the plurality of comparable users, the comparable feature matrices being generated based on the comparable feature vectors and comparable training target vectors of the plurality of comparable users.

In some embodiments, the machine learning model is trained using the comparable feature vectors as inputs and the comparable training target vectors as outputs.

In some embodiments, the recommendation unit determines a preference matrix based on comparable users' preferences for different fitness effect information; determines a match matrix based on degrees of match between fitness effect information and different fitness regimen information; determines degrees of recommendation for different types of fitness regimen information suggested by the comparable users based on the preference matrix and the match matrix; determines the fitness regimen information of the user to be recommended based on a degree of recommendation of a comparable user associated with the matching human body model selected by the user to be recommended.

According to some still other embodiments of the present disclosure, there is provided A recommendation apparatus for fitness regimen information, comprising: a memory; a processor coupled to the memory, the processor configured to, based on instructions stored in the memory, carry out the fitness regimen information recommendation method according to any one of the above embodiments.

According to still other embodiments of the present disclosure, there is provided a non-transitory computer readable storage medium having stored thereon a computer program that, when executed by a processor, implements the fitness regimen information recommendation method according to any one of the above embodiments.

## BRIEF DESCRIPTION OF THE DRAWINGS

The accompanying drawings, which are incorporated in and constitute a portion of this specification, illustrate embodiments of the present disclosure and, together with the description, serve to explain the principles of the present disclosure.

The present disclosure will be more clearly understood from the following detailed description with reference to the accompanying drawings, in which:

FIG. 1 shows a flowchart of a recommendation method for fitness regimen information according to some embodiments of the present disclosure;
FIG. 2 shows a flowchart of step 110 in FIG. 1 according to some embodiments of the present disclosure;
FIG. 3 shows a flowchart of a recommendation method for fitness regimen information according to other embodiments of the present disclosure;
FIG. 4 shows a block diagram of a recommendation apparatus for fitness regimen information according to some embodiments of the present disclosure;
FIG. 5 shows a block diagram of a recommendation apparatus for fitness regimen information according to other embodiments of the present disclosure;
FIG. 6 shows a block diagram of a recommendation apparatus for fitness regimen information according to further embodiments of the present disclosure.

## DETAILED DESCRIPTION

Various exemplary embodiments of the present disclosure will now be described in detail with reference to the accompanying drawings. Notice that, unless otherwise specified, the relative arrangement, numerical expressions and numerical values of the components and steps set forth in these examples do not limit the scope of the invention.

At the same time, it should be understood that, for ease of description, the dimensions of the various parts shown in the drawings are not drawn to actual proportions.

The following description of at least one exemplary embodiment is in fact merely illustrative and is in no way intended as a limitation to the invention, its application or use.

Techniques, methods, and apparatus known to those of ordinary skill in the relevant art may not be discussed in detail, but where appropriate, these techniques, methods, and apparatuses should be considered as part of the specification.

Of all the examples shown and discussed herein, any specific value should be construed as merely illustrative and not as a limitation. Thus, other examples of exemplary embodiments may have different values.

Notice that, similar reference numerals and letters are denoted by the like in the accompanying drawings, and therefore, once an item is defined in a drawing, there is no need for further discussion in the accompanying drawings.

As mentioned above, recommending fitness regimen information based on a general model does not take into account differences between users. For example, the differences may comprise differences in muscle types, differences in dietary habits, and so on. With fitness regimen information recommended based on the general model, different effects may be achieved for different users, and some users may not be able to achieve the desired results despite their best efforts, resulting in poor user experience.

For example, a general model cannot accurately describe different abdominal muscles shapes. In the case where the desired training effect is to achieve one of the abdominal muscles shapes, the recommended fitness regimen information based on the general model may not enable the user to achieve the desired abdominal muscles shape.

In response to the above technical problems, this disclosure matches a user's information (such as a user human body model, etc.) with comparable users to determine a matching user; based on the user's training part, training intensity and training effect, etc., fitness regimen information is recommended for the user according to the matching user's training intensity and fitness regimen, etc. In this way, the user's ability to achieve the training effect can be improved.

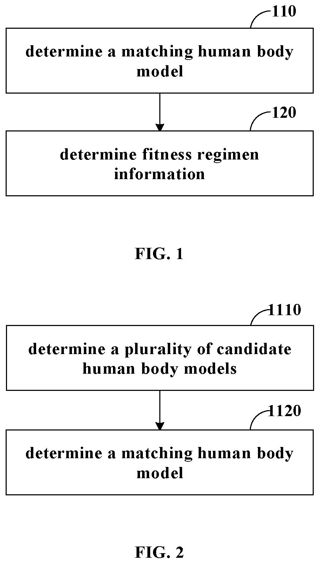

FIG. 1 shows a flowchart of a recommendation method for fitness regimen information according to some embodiments of the present disclosure.

As shown in FIG. 1, in step 110, based on physiological feature information of a user to be recommended, a matching human body model for the physiological feature information is determined from a plurality of comparable human body models. The plurality of comparable human body models are generated based on physiological feature information of a plurality of comparable users.

In some embodiments, a fitness database comprising a plurality of comparable human body models and related information of the plurality of comparable human body models can be built in advance. A matching human body model can be determined from the fitness database based on the physiological feature information of the user to be recommended.

For example, the system obtains physiological feature information input by comparable users such as fitness models, fitness coaches, and other users willing to share data. The physiological feature information may comprise height, weight, body fat, etc. The system can also obtain image information of the comparable users, such as full-body 2D photos or partial body photos.

For example, the system constructs comparable human body models based on the physiological feature information input by the comparable users. For example, the human body model may be a digital human body models 3D human body model, etc. The comparable human body models may be human body models constructed based on physiological feature information such as height, weight, and body fat.

For example, the system can regularly obtain physiological feature information and fitness regimen information of comparable users, and generate comparable human body models; the physiological feature information, fitness regimen information and the comparable human body models of the comparable users can be related and stored in the fitness database. The fitness regimen information of the comparable users may comprise training time, training effect, training intensity, training period, etc.

For example, the fitness regimen information, training effect, and other relevant information of comparable users can be stored on a blockchain network.

In some embodiments, a user human body model of the user to be recommended is generated based on physiological feature information of the user to be recommended; a matching human body model is determined based on a degree of similarity between the user human body model and a plurality of comparable human body models.

In some embodiments, a user current human body model of the user to be recommended is generated based on physiological feature information of the user to be recommended; a user target human body model is generated according to an adjustment of the user to be recommended for the user current human body model based on a fitness target; a matching human body model is determined based on a degree of similarity between the user target human body model and a plurality of comparable human body models.

For example, a current human body model of the user to be recommended is generated based on physiological feature information acquired from the user to be recommended; the user to be recommended can perform an adjustment operation on all or at least one part of the current human body model to change the current human body model to generate a target human body model.

For example, the adjustment operation may be clicking on adjustment buttons, such as plus or minus buttons for the current human body model, or direct drag-and-drop to enlarge or shrink the model.

In some embodiments, a comparable human body model with an overlap area greater than a threshold value with the user human body model is determined as a matching human body model. For example, the overlap area can be determined by one of the following methods.

For example, 2D images of a comparable human body model and the user human body model can be overlapped to calculate the overlap area of the images.

For example, the overlap area can be determined based on a surface area ratio between the user human body model and different 3D human body models. In this way, the overlap area can be determined by a surface area ratio between 3D data maps, which is easier to implement.

For example, if the volume of the user human body model is smaller than the volumes of the comparable human body models, a comparable human body model with the smallest volume of the comparable human body models is determined as the matching human body model; if the volume of the user human body model is greater than the volumes of the comparable human body models, a comparable human body model with the largest volume of the comparable human body models is determined as the matching human body model. In this way, a more accurate matching human body model can be determined.

In some embodiments, the volume of the user human body model is smaller than that of any comparable human body model, but a particular part of the user human body model (such as the forearm) is larger than the particular part of a comparable human body model, and another part (such as the upper arm) is smaller than the other part of a comparable human body model. In this case, the user human body model can be proportionally scaled first to improve the accuracy of determining the matching human body model.

For example, the user human body model can be proportionally scaled to generate a scaled user human body model which matches the size of the comparable human body models; a matching human body model is determined based on a degree of similarity between the scaled user human body model and the comparable human body models.

In some embodiments, the current human body model is matched with comparable human body models in a fitness database; two human body models are considered to match if their overlap area is greater than a threshold value.

In some embodiments, a target human body model is matched with comparable human body models in a fitness database; two human body models are considered to match if their overlap area is greater than a threshold value.

In some embodiments, the current human body model is proportionally scaled and then is matched with comparable human body models in a fitness database; two 3D models are considered to match if their overlap area is greater than a threshold value.

In some embodiments, a target human body model is proportionally scaled and then is matched with comparable human body models in a fitness database; two human body models are considered to match if their overlap area is greater than a threshold value.

In some embodiments, the system obtains physiological feature information of the user to be recommended. For example, the system can obtain image information of the user to be recommended, such as full-body 2D photos or partial body photos.

For example, the system matches comparable human body models of comparable users in a fitness database using physiological feature information (comprising at least one of height, weight, BWH, BFP) of the user to be recommended.

For example, the system can construct a user human body model of the user to be recommended based on physiological feature information of the user to be recommended, match the user human body model with comparable human body models in a fitness database, and determine a comparable human body model similar to the user human body model as a matching human body model, and recommend fitness regimen information related to the matching human body model to the user to be recommended.

This allows for more targeted fitness recommendations to users.

In some embodiments, due to non-linear body fat loss, i.e. different rates of body fat loss at different stages, a matching human body model can be determined based on body fat information of the user to be recommended.

For example, based on physiological feature information such as height, weight, and total body fat information of the user to be recommended, a matching human body model is determined in the fitness database, and then a comparable user corresponding to the matching human body model is determined as a matching user. Alternatively, a matching human body model can be determined from the fitness database based on the physiological feature information such as height, weight, and body fat information of one or more body parts of the user to be recommended.

In some embodiments, a matching human body model can also be determined based on physiological feature information other than body fat, such as height, weight, and BWH of the user to be recommended.

In some embodiments, the user human body model is matched with comparable human body models in a fitness database; two human body models are considered to match if their overlap area is greater than a threshold value.

In some embodiments, the user human body model is proportionally scaled and then is matched with comparable human body models in a fitness database; two human body models are considered to match if their overlap area is greater than a threshold value.

In this way, by matching human body models, a comparable user is identified which is in the same or similar situation as the user to be recommended, resulting in more targeted recommended fitness regimen information.

In some embodiments, a user feature vector is generated based on the physiological feature information of the user to be recommended; a matching user is determined from a plurality of comparable users based on a degree of similarity between the user feature vector and comparable feature vectors of the plurality of comparable users, the comparable feature vectors being generated based on physiological feature information of the comparable users; a comparable human body model of the matching user is determined as the matching human body model.

For example, a degree of similarity between users is calculated through a user-based collaborative filtering recommendation algorithm. The user-based collaborative filtering recommendation algorithm can be implemented by calculating cosine similarity. The feature vectors of different users are as follows:

| User | Height | Weight | Body Fat |
|------|--------|--------|----------|
| User A | A1 | A2 | A3 |
| User B | B1 | B2 | B3 |
| User C | C1 | C2 | C3 |

The user to be recommended is user A, the comparable user is user B, and a degree of similarity between user A and user B can be expressed as a cosine value between two feature vectors:

$$(A1 \times B1 + A2 \times B2 + A3 \times B3)/\{(A1^2 + A2^2 + A3^2) + (B1^2 + B2^2 + B3^2)\}$$

In some embodiments, a user feature vector is generated based on at least one of living habit information of the user to be recommended and a weight of the living habit information, health condition information of the user to be recommended and a weight of the health condition information, as well as the physiological feature information of the user to be recommended and a weight of the physiological feature information; a comparable feature vector is generated based on at least one of living habit information of the comparable user and a weight of the living habit information, health condition information of the comparable user and a weight of the health condition information, as well as physiological feature information of the comparable user and a weight of the physiological feature information.

In some embodiments, the weight of the health condition information of the user to be recommended is greater than the weight of the living habit information of the user to be recommended, and the weight of the living habit information of the user to be recommended is greater than the weight of the physiological feature information of the user to be recommended; the weight of the health condition information of the comparable user is greater than the weight of the living habit information of the comparable user, and the weight of living habit information of the comparable user is greater than the weight of the physiological feature information of the comparable user.

For example, the feature vectors of different users are as follows:

| User | Height | Weight | Body Fat | Dietary Habits | Food Preferences | Rest Habits |
|---|---|---|---|---|---|---|
| User A | A1 | A2 | A3 | A4 | A5 | A6 |
| User B | B1 | B2 | B3 | B4 | B5 | B6 |
| User C | C1 | C2 | C3 | C4 | C5 | C6 |

Due to the varying influence of different information on subsequent training effects, different weights can be assigned to different information in the cosine similarity calculation. For example, feature vectors of different users are obtained as follows by weighting the above information:

| User | Height | Weight | Body Fat | Dietary Habits | Food Preferences | Rest Habits |
|---|---|---|---|---|---|---|
| User A | A1 | A2 | A3 | A4×1.5 | A5×1.5 | A6 |
| User B | B1 | B2 | B3 | B4×1.5 | B5×1.5 | B6 |
| User C | C1 | C2 | C3 | C4×1.5 | C5×1.5 | C6 |

For example, the feature vectors of different users are as follows:

| User | Height | Weight | Body Fat | Dietary Habits | Food Preferences | Rest Habits | Heart Rate | Lung Capacity |
|---|---|---|---|---|---|---|---|---|
| User A | A1 | A2 | A3 | A4 | A5 | A6 | A7 | A8 |
| User B | B1 | B2 | B3 | B4 | B5 | B6 | B7 | B8 |
| User C | C1 | C2 | C3 | C4 | C5 | C6 | C7 | C8 |

For example, physiological feature information (such as BWH parameters), living habit information (such as dietary related parameters), and health condition information have different weights q. Health condition information has the highest weight, living habit information has the second highest weight, and physiological feature information has the lowest weight, as shown below.

| User | Height | Weight | Body Fat | Dietary Habits | Food Preferences | Rest Habits | Heart Rate | Lung Capacity |
|---|---|---|---|---|---|---|---|---|
| User A | A1 | A2 | A3 | A4×q | A5×q | A6 | A7×p | A8×p |
| User B | B1 | B2 | B3 | B4×q | B5×q | B6 | B7×p | B8×p |

(continued)

| User | Height | Weight | Body Fat | Dietary Habits | Food Preferences | Rest Habits | Heart Rate | Lung Capacity |
|---|---|---|---|---|---|---|---|---|
| User C | C1 | C2 | C3 | C4×q | C5×q | C6 | C7×p | C8×p |

For example, different contents of the same type of information can have different weights. For example, the weight of heart rate may be greater than the weight of lung capacity.

In some embodiments, using a machine learning model, a user training target vector of the user to be recommended is determined based on the user feature vector, the user training target vector comprising training target information corresponding to a body part which the user to be recommended wants to achieve; a user feature matrix of the user to be recommended is determined based on the user feature vector and the user training target vector; a matching user is determined from a plurality of comparable users based on a degree of similarity between user feature matrix and comparable feature matrices of the plurality of comparable users, the comparable feature matrices being generated based on the comparable feature vectors and comparable training target vectors of the plurality of comparable users.

In some embodiments, the machine learning model is trained using the comparable feature vectors as inputs and the comparable training target vectors as outputs.

For example, a neural network model can be constructed as the machine learning model. The neural network model is trained using the data from a fitness database, comprising height, weight, body fat, etc., as inputs to the neural network model, and the fitness regimen information selected by the comparable users as outputs. Information about dietary habits, food preferences, sleep habits, and health-related information may also be used as inputs. A fitness database can be built for comparable users, comprising height, weight, body fat, dietary habits, food preferences, rest habits, heart rate, lung capacity, and fitness regimens:

| User | Height | Weight | Body Fat | Dietary Habits | Food Preferences | Rest Habits | Heart Rate | Lung Capacity | Fitness regimen |
|---|---|---|---|---|---|---|---|---|---|
| User A | A1 | A2 | A3 | A4 | A5 | A6 | A7 | A8 | X |
| User B | B1 | B2 | B3 | B4 | B5 | B6 | B7 | B8 | Y |
| User C | C1 | C2 | C3 | C4 | C5 | C6 | C7 | C8 | Z |

A feature vector U and a comparable training target vector E of a comparable user are obtained. For example, the comparable feature vector U is as follows:

| User | Height | Weight | Body Fat | Dietary Habits | Food Preferences | Rest Habits | Heart Rate | Lung Capacity |
|---|---|---|---|---|---|---|---|---|
| User A | A1 | A2 | A3 | A4 | A5 | A6 | A7 | A8 |
| User B | B1 | B2 | B3 | B4 | B5 | B6 | B7 | B8 |
| User C | C1 | C2 | C3 | C4 | C5 | C6 | C7 | C8 |

The comparable training target vector E is as follows:

| Fitness regimen | biceps | triceps | deltoids | forearms | abdominal muscles | dorsal muscles | gluteus | thighs | calves |
|---|---|---|---|---|---|---|---|---|---|
| X | X1 | X2 | X3 | X4 | X5 | X6 | X7 | X8 | X9 |
| Y | Y1 | Y2 | Y3 | Y4 | Y5 | Y6 | Y7 | Y8 | Y9 |
| Z | Z1 | Z2 | Z3 | Z4 | Z5 | Z6 | Z7 | Z8 | Z9 |

X1~X9, Y1~Y9, Z1~Z9 may be the shape, circumference, partial BFP of the corresponding part.

By multiplying U and E, a comparable feature matrix $R=U\times E$ between the user information and the fitness regimen information is obtained as follows:

|  | biceps | triceps | deltoids | forearms | abdominal muscles | dorsal muscles | gluteus | thighs | calves |
|---|---|---|---|---|---|---|---|---|---|
| Height | R11 | R12 | R13 | R14 | R15 | R16 | R17 | R18 | R19 |
| Weight | R21 | R22 | R23 | R24 | R25 | R26 | R27 | R28 | R29 |
| Body Fat | R31 | R32 | R33 | R34 | R35 | R36 | R37 | R38 | R39 |
| Dietary Habits | R41 | R42 | R43 | R44 | R45 | R46 | R47 | R48 | R49 |
| Food Preferences | R51 | R52 | R53 | R54 | R55 | R56 | R57 | R58 | R59 |
| Rest Habits | R61 | R62 | R63 | R64 | R65 | R66 | R67 | R68 | R69 |
| Heart Rate | R71 | R72 | R73 | R74 | R75 | R76 | R77 | R78 | R79S |
| Lung Capacity | R81 | R82 | R83 | R84 | R85 | R86 | R87 | R88 | R89 |

Fitness regimen information recommended can be determined via the neural network model for the user to be recommended:

| User | Height | Weight | Body Fat | Dietary Habits | Food Preferences | Rest Habits | Heart Rate | Lung Capacity | Fitness regimen |
|------|--------|--------|----------|----------------|------------------|-------------|------------|---------------|-----------------|
| User A | A1 | A2 | A3 | A4 | A5 | A6 | A7 | A8 | X |
| User B | B1 | B2 | B3 | B4 | B5 | B6 | B7 | B8 | Y |
| User C | C1 | C2 | C3 | C4 | C5 | C6 | C7 | C8 | Z |
| User D | D1 | D2 | D3 | D4 | D5 | D6 | D7 | D8 | X |

For example, fitness regimen information can be recommended for the user to be recommended according to R of the user to be recommended.

In step 120, fitness regimen information is determined for the user to be recommended based on fitness regimen information of a comparable user associated with the matching human body model.

In some embodiments, fitness effect information of the comparable user associated with the matching human body model and corresponding fitness regimen information of the fitness effect information are displayed to the user to be recommended. Fitness regimen information of the user to be recommended is determined based on a selection of the fitness effect information and the corresponding fitness regimen information by the user to be recommended.

For example, a human body model corresponding to the fitness effect information can be displayed to the user to be recommended, allowing the user to more intuitively select the desired fitness effect, thereby improving the accuracy of the recommendation. In some embodiments, according to a target part selected by the user to be recommended, a plurality of fitness effect information of the target part associated with the matching human body model, as well as the training period and training intensity corresponding to the each of the plurality of fitness effect information, are displayed to the user to be recommended; based on target fitness effect information selected from the plurality of fitness effect information by the user to be recommended, a training period and a training intensity corresponding to the target fitness effect information are determined as the fitness regimen information.

In some embodiments, a training period and a training intensity desired by the user to be recommended can be determined first, and then a fitness effect corresponding to the matching human body model can be displayed to the user to be recommended.

For example, a selection for a target part and a training intensity is received from the user to be recommended; fitness regimen information is then determined for the user to be recommended based on fitness regimen information and an training effect of the target part associated with a matching human body model, as well as the training intensity selected by the user to be recommended.

For example, a plurality of users who are training the same target part can be identified; the user to be recommended is prompted in real time with current score and/or ranking of the user among the plurality of users.

In some embodiments, fitness effects, training periods, and training intensities associated with a matching human body model may be shown first, and then determine the recommended fitness regimen information based on the selection of the user to be recommended.

For example, a selection for the target part is received from the user to be recommended; various types of fitness regimen information and training effects (which can be intermediate training effects before achieving the training target) of the target part associated with a matching human body model are obtained; a selection for an training effect is received from the user to be recommended; and fitness regimen information associated with selected training effect is recommended to the user to be recommended.

In some embodiments, a preference matrix is determined based on preferences of a comparable user for different fitness effect information.

For example, taking fitness effect information related to forearm as an example, preferences of comparable users User 1 to User 3 for different fitness effect information can be determined based on the selection of the comparable users for fitness effect information such as Massive forearms, Bigger forearms, Ordinary forearms, and Skinny forearms; a preference matrix is then constructed based on the preferences as shown in Table 1:

Table 1

| | Class1 Massive forearms | Class2 Bigger forearms | Class3 Ordinary forearms | Class4 Skinny forearms |
|---|---|---|---|---|
| User 1 (Zhang San) | 1 | 0 | 0 | 0 |
| User2 (Li Si) | 0 | 1 | 0 | 0 |
| User3 (Wang Wu) | 0 | 0 | 0 | 1 |

In the case of mutual exclusion between different fitness effect information, a preference may be 1 or 0 representing Prefer or Not Prefer, respectively; in the absence of mutual exclusion between different fitness effect information, a preference can be represented by a value ranging from 0 to 1, the larger the value, the higher the preference.

In some embodiments, a match matrix is determined based on the degree of match between fitness effect information and different fitness regimen information. For example, a match matrix can be constructed as shown in Table 2:

Table 2

| | Item 1 (Fitness regimen 1) | Item 2 (Fitness regimen 2) | Item 3 (Fitness regimen 3) | Item 4 (Fitness regimen 4) |
|---|---|---|---|---|
| Class 1 (Massive forearms) | Match Degree (0.9) | Match Degree (0.2) | Match Degree (0.1) | Match Degree (0.4) |
| Class 2 (Bigger forearms) | Match Degree (0.6) | Match Degree (0.7) | Match Degree (0.2) | Match Degree (0.3) |
| Class 3 (Ordinary forearms) | Match Degree (0.7) | Match Degree (0.4) | Match Degree (0.3) | Match Degree (0.2) |
| Class 4 (Skinny forearms) | Match Degree (0.2) | Match Degree (0.3) | Match Degree (0.4) | Match Degree (0.1) |

The matching degree may be a value between 0 and 1, and the larger the value, the higher the match degree.

In some embodiments, degrees of recommendation of the comparable users for different types of fitness regimen information are determined based on the preference matrix and the match matrix. For example, a recommendation matrix can be determined based on the product of the preference matrix and the match matrix. The recommendation matrix comprises degrees of recommendation of different comparable users for different fitness regimens, as shown in Table 3:

Table 3

| | Item 1 (Fitness regimen 1) | Item 2 (Fitness regimen 2) | Item 3 (Fitness regimen 3) | Item 4 (Fitness regimen 4) |
|---|---|---|---|---|
| User 1 (Zhang San) | 0.9 | 0.2 | 0.1 | 0.4 |
| User 2 (Li Si) | 0.6 | 0.7 | 0.2 | 0.3 |
| User 3 (Wang Wu) | 0.2 | 0.3 | 0.4 | 0.1 |

In some embodiments, the degrees of recommendation of the comparable users associated with the matching human body model for different fitness regimens are present to the user to be recommended; the user to be recommended can select his/her own fitness regimen information based on degrees of recommendation.

In the above embodiment, the correlation between fitness effects and fitness regimens can be established through the preference matrix and the match matrix, thereby providing the degrees of recommendation of the comparable users for different fitness regimens; the user to be recommended can select a desired fitness regimen based on a matching comparable user, thereby overcoming the problem of decreased recommendation effectiveness caused by sparse user data.

FIG. 2 shows a flowchart of step 110 in FIG. 1 according to some embodiments of the present disclosure.

As shown in FIG. 2, in step 1110, a plurality of candidate human body models are determined.

In some embodiments, taking into account different living habits, such as the ingestion ability of the users, a matching human body model is determined from the plurality of comparable human body models based on the physiological feature information and the living habit information of the user to be recommended. For example, the living habit information may comprise at least one of dietary habits, food preferences, and rest habits.

In some embodiments, the system obtains living habit information of comparable users, and stores the physiological feature information, fitness regimen information, living habit information of the comparable users in association with comparable human body models in a fitness database.

For example, a plurality of candidate human body models are determined from a plurality of comparable human body models based on physiological feature information of the user to be recommended.

For example, a plurality of candidate human body models are determined from a plurality of comparable human body models based on the living habit information of the user to be recommended.

In some embodiments, a matching human body model is also determined from the plurality of comparable human body models based on health condition information of the user to be recommended. For example, the health condition information may comprise at least one of heart rate, blood pressure, and blood glucose.

In some embodiments, the system obtains health condition information of comparable users, and stores the physiological feature information, fitness regimen information, living habit information, and health condition information of the comparable users in association with comparable human body models in a fitness database.

For example, a plurality of candidate human body models are determined from a plurality of comparable human body models based on the living habit information and the health condition information of the user to be recommended.

In step 1120, a matching human body model is determined.

In some embodiments, a matching human body model can be determined from the plurality of candidate human body models based on living habit information of the user to be recommended.

In some embodiments, a matching human body model is determined from the plurality of candidate human body models based on physiological feature information of the user to be recommended.

In some embodiments, a matching human body model is determined from the plurality of candidate human body models based on the living habit information and the health condition information of the user to be recommended.

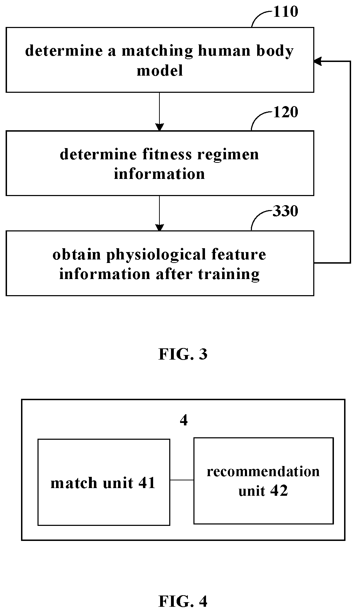

FIG. 3 shows a flowchart of a recommendation method for fitness regimen information according to other embodiments of the present disclosure.

As shown in FIG. 3, the recommendation method may comprise step 330.

In step 330, physiological feature information after training of the user to be recommended is obtained after training for a preset period of time based on the fitness regimen information.

Then, steps 110 and 120 are repeated. A human body model after training is generated based on the physiological feature information after training. A matching human body model for the human body model after training is determined from a plurality of comparable human body models based on the human body model after training; new fitness regimen information is then determined for the user to be recommended based on fitness regimen information associated with the matching human body model for the human body model after training.

FIG. 4 shows a block diagram of A recommendation apparatus for fitness regimen information according to some embodiments of the present disclosure.

As shown in FIG. 4, the apparatus 4 for recommending fitness regimen information comprises a match unit 41 and a recommendation unit 42.

The match unit 41 determines, based on physiological feature information of a user to be recommended, a matching human body model for the physiological feature information from a plurality of comparable human body models, the plurality of comparable human body models being generated based on physiological feature information of a plurality of comparable users; the recommendation unit 42 determines fitness regimen information for the user to be recommended based on fitness regimen information of a comparable user associated with the matching human body model.

In some embodiments, the recommendation unit 42 displays fitness effect information of the comparable user associated with the matching human body model and corresponding fitness regimen information of the fitness effect information to the user to be recommended; determines fitness regimen information of the user to be recommended based on a selection of the fitness effect information and the corresponding fitness regimen information by the user to be recommended.

In some embodiments, the recommendation unit 42 displays, according to a target part selected by the user to be recommended, a plurality of fitness effect information of the target part associated with the matching human body model, training periods and training intensities corresponding to the plurality of fitness effect information, to the user to be recommended; determines, based on target fitness effect information selected from the plurality of fitness effect information by the user to be recommended, a training period and a training intensity corresponding to the target fitness effect information as the fitness regimen information.

In some embodiments, the match unit 41 generates a user human body model of the user to be recommended based on the physiological feature information of the user to be recommended; determines a matching human body model based on a degree of similarity between the user human body model and a plurality of comparable human body models.

In some embodiments, the match unit 41 determines a comparable human body model with an overlap area greater than a threshold value with the user human body model as a matching human body model.

In some embodiments, the match unit 41 proportionally scales the user human body model to generate a scaled user human body model which matches the size of the comparable human body models; determines a matching human body model based on a degree of similarity between the scaled user human body model and the comparable human body models.

In some embodiments, the match unit 41 generates a user current human body model of the user to be recommended based on the physiological feature information of the user to be recommended; the match unit 41 generates a user target human body model according to an adjustment of the user to be recommended for the user current human body model based on a fitness target; the match unit 41 determines the matching human body model based on a degree of similarity between the user target human body model and the plurality of comparable human body models.

In some embodiments, the match unit 41 determines the matching human body model from the plurality of comparable human body models based on the physiological feature information and living habit information of the user to be recommended.

In some embodiments, the match unit 41 determines a plurality of candidate human body models from a plurality of comparable human body models based on the physiological feature information of the user to be recommended; determines the matching human body model from the plurality of candidate human body models based on the living habit information of the user to be recommended; or determines a plurality of candidate human body models from the plurality of comparable human body models based on the living habit information of the user to be recommended; determines the matching human body model from the plurality of candidate human body models based on the physiological feature information of the user to be recommended.

In some embodiments, the match unit 41 determines a matching human body model from the plurality of comparable human body models, also based on health condition information of the user to be recommended.

In some embodiments, the match unit 41 determines a plurality of candidate human body models from a plurality of comparable human body models based on the physiological feature information of the user to be recommended; determines the matching human body model from the plurality of candidate human body models based on the living habit information and the health condition information of the user to be recommended; or determines a plurality of candidate human body models from the plurality of comparable human body models based on the living habit information and the health condition information of the user to be recommended; determines the matching human body model from the plurality of candidate human body models based on the physiological feature information of the user to be recommended.

In some embodiments, the match unit 41 acquires physiological feature information after training of the user to be recommended after training for a preset period of time based on the fitness regimen information; generates a human body model after training based on the physiological feature information after training; determines a matching human body model for the human body model after training from the plurality of comparable human body models based on the human body model after training; the recommendation unit 42 determines new fitness regimen information for the user to be recommended based on fitness regimen information associated with the matching human body model for the human body model after training.

In some embodiments, the match unit 41 generates a user feature vector based on the physiological feature information of the user to be recommended; determines a matching user from a plurality of comparable users based on a degree of similarity between the user feature vector and comparable feature vectors of the plurality of comparable users, the comparable feature vectors being generated based on physiological feature information of the comparable users; determines the comparable human body model of the matching user as the matching human body model.

In some embodiments, the match unit 41 generates a user feature vector based on at least one of living habit information of the user to be recommended and a weight of the living habit information, health condition information of the user to be recommended and a weight of the health condition information, as well as the physiological feature information of the user to be recommended and a weight of the physiological feature information; and generates a comparable feature vector based on at least one of living habit information of the comparable user and a weight of the living habit information, health condition information of the comparable user and a weight of the health condition information, as well as physiological feature information of the comparable user and a weight of the physiological feature information.

In some embodiments, the weight of the health condition information of the user to be recommended is greater than the weight of the living habit information of the user to be recommended, and the weight of the living habit information of the user to be recommended is greater than the weight of the physiological feature information of the user to be recommended; the weight of the health condition information of the comparable user is greater than the weight of the living habit information of the comparable user, and the weight of living habit information of the comparable user is greater than the weight of the physiological feature information of the comparable user.

In some embodiments, the match unit 41 determines, using a machine learning model, a user training target vector of the user to be recommended based on the user feature vector, the user training target vector comprising training target information corresponding to a body part which the user to be recommended wants to achieve; determines a user feature matrix of the user to be recommended based on the user feature vector and the user training target vector; determines a matching user from a plurality of comparable users based on a degree of similarity between user feature matrix and comparable feature matrices of the plurality of comparable users, the comparable feature matrices being generated based on the comparable feature vectors and comparable training target vectors of the plurality of comparable users.

In some embodiments, the machine learning model is trained using the comparable feature vectors as inputs and the comparable training target vectors as outputs.

In some embodiments, the recommendation unit 42 determines a preference matrix based on comparable users' preferences for different fitness effect information; determines a match matrix based on degrees of match between fitness effect information and different fitness regimen information; determines degrees of recommendation for different types of fitness regimen information suggested by the comparable users based on the preference matrix and the match matrix; determines the fitness regimen information of the user to be recommended based on a degree of recommendation of a comparable user associated with the matching human body model selected by the user to be recommended.

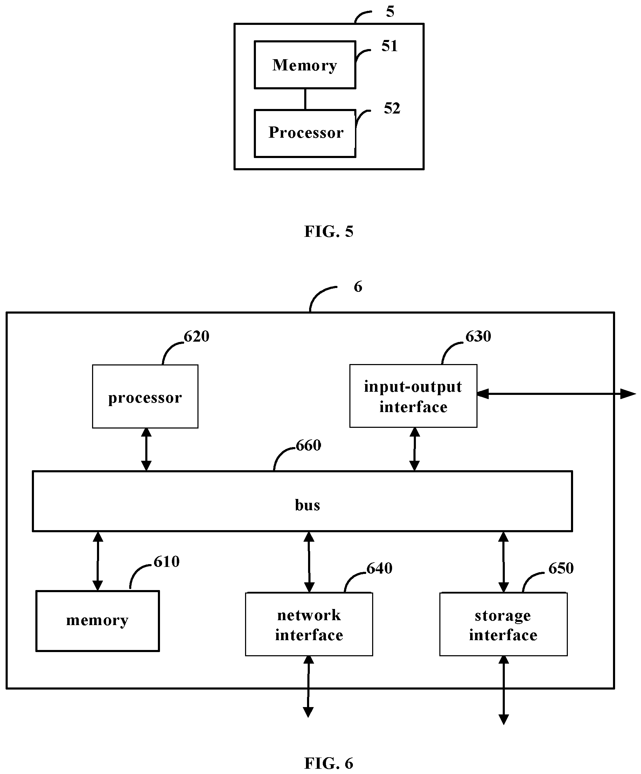

FIG. 5 shows a block diagram of A recommendation apparatus for fitness regimen information according to other embodiments of the present disclosure.

As shown in FIG. 5, the apparatus 5 for recommending fitness regimen information of this embodiment comprises: a memory 51 and a processor 52 coupled to the memory 51, the processor 52 configured to, based on instructions stored in the memory 51, carry out the recommendation method for fitness regimen information according to any one of the embodiments of the present disclosure.

Wherein, the memory 51 may comprise, for example, system memory, a fixed non-transitory storage medium, or the like. The system memory stores, for example, an operating system, applications, a boot loader, a database, and other programs.

FIG. 6 shows a block diagram of A recommendation apparatus for fitness regimen information according to further embodiments of the present disclosure.

As shown in FIG. 6, the apparatus 6 for recommending fitness regimen information of this embodiment comprises: a memory 610 and a processor 620 coupled to the memory 610, the processor 620 configured to, based on instructions stored in the memory 610, carry out the recommendation method for fitness regimen information according to any one of the embodiments of the present disclosure.

The memory 610 may comprise, for example, system memory, a fixed non-transitory storage medium, or the like. The system memory stores, for example, an operating system, application programs, a boot loader, and other programs.

The apparatus 6 for recommending fitness regimen information may further comprise an input-output interface 630, a network interface 640, a storage interface 650, and the like. These interfaces 630, 640, 650, the memory 610 and the processor 620 may be connected through a bus 660, for example. Wherein, the input-output interface 630 provides a connection interface for input-output devices such as a display, a mouse, a keyboard, a touch screen, a microphone, a loudspeaker, etc. The network interface 640 provides a connection interface for various networked devices. The storage interface 650 provides a connection interface for external storage devices such as an SD card and a USB flash disk.

Those skilled in the art should understand that the embodiments of the present disclosure may be provided as a method, a system, or a computer program product. Therefore, embodiments of the present disclosure can take the form of an entirely hardware embodiment, an entirely software embodiment or an embodiment comprising both hardware and software elements. Moreover, the present disclosure may take the form of a computer program product embodied on one or more computer-usable non-transitory storage media (comprising but not limited to disk storage, CD-ROM, optical memory, etc.) having computer-usable program code embodied therein.

Heretofore, the method and recommendation apparatus for fitness regimen information and the non-transitory computer-readable storage medium according to the present disclosure have been described in detail. In order to avoid obscuring the concepts of the present disclosure, some details known in the art are not described. Based on the above description, those skilled in the art can understand how to implement the technical solutions disclosed herein.

The method and system of the present disclosure may be implemented in many ways. For example, the method and system of the present disclosure may be implemented by software, hardware, firmware, or any combination of software, hardware, and firmware. The above sequence of steps of the method is merely for the purpose of illustration, and the steps of the method of the present disclosure are not limited to the above-described specific order unless otherwise specified. In addition, in some embodiments, the present disclosure may also be implemented as programs recorded in a recording medium, which comprise machine-readable instructions for implementing the method according to the present disclosure. Thus, the present disclosure also covers a recording medium storing programs for executing the method according to the present disclosure.

Although some specific embodiments of the present disclosure have been described in detail by way of example, those skilled in the art should understand that the above examples are only for the purpose of illustration and are not intended to limit the scope of the present disclosure. It should be understood by those skilled in the art that the above embodiments may be modified without departing from the scope and spirit of the present disclosure. The scope of the disclosure is defined by the following claims.

**Claims**

1. A recommendation method for fitness regimen information, comprising:

   determining, based on physiological feature information of a user to be recommended, a matching human body model for the physiological feature information from a plurality of comparable human body models, the plurality of comparable human body models being generated based on physiological feature information of a plurality of comparable users; and
   determining fitness regimen information for the user to be recommended based on fitness regimen information of a comparable user associated with the matching human body model.

2. The recommendation method according to claim 1, wherein the determining fitness regimen information for the user to be recommended based on the fitness regimen information of the comparable user associated with the matching human body model comprises:

   displaying fitness effect information of the comparable user associated with the matching human body model and corresponding fitness regimen information of the fitness effect information to the user to be recommended; and
   determining the fitness regimen information of the user to be recommended based on a selection of the fitness effect information and the corresponding fitness regimen information by the user to be recommended.

3. The recommendation method according to claim 1, wherein the determining fitness regimen information for the user to be recommended based on the fitness regimen information of the comparable user associated with the matching human body model comprises:

   displaying a plurality of fitness effect information of a target part associated with the matching human body model to the user to be recommended, training periods and training intensities corresponding to the plurality of fitness effect information, according to the target part selected by the user to be recommended; and
   determining, based on target fitness effect information selected from the plurality of fitness effect information by the user to be recommended, a training period and a training intensity corresponding to the target fitness effect information as the fitness regimen information.

4. The recommendation method according to claim 1, wherein the determining, based on the physiological feature information of the user to be recommended, the matching human body model for the physiological feature information from the plurality of comparable human body models comprises:

   generating a user human body model of the user to be recommended based on the physiological feature information of the user to be recommended; and
   determining the matching human body model based on a degree of similarity between the user human body model and the plurality of comparable human body models.

**5.** The recommendation method according to claim 4, wherein the determining the matching human body model based on the degree of similarity between the user human body model and the plurality of comparable human body models comprises:
determining a comparable human body model with an overlap area greater than a threshold value with the user human body model as the matching human body model.

**6.** The recommendation method according to claim 4, wherein the determining the matching human body model based on the degree of similarity between the user human body model and the plurality of comparable human body models comprises:

proportionally scaling the user human body model to generate a scaled user human body model which matches the size of the plurality of comparable human body models; and
determining a matching human body model based on the degree of similarity between the scaled user human body model and the comparable human body models.

**7.** The generation method according to claim 4, wherein the generating the user human body model of the user to be recommended based on the physiological feature information of the user to be recommended comprises:

generating a user current human body model of the user to be recommended based on the physiological feature information of the user to be recommended; and
generating a user target human body model according to an adjustment of the user to be recommended for the user current human body model based on a fitness target;
the determining the matching human body model based on the degree of similarity between the user human body model and the plurality of comparable human body models comprises:
determining the matching human body model based on the degree of similarity between the user target human body model and the plurality of comparable human body models.

**8.** The recommendation method according to claim 1, wherein the determining, based on the physiological feature information of the user to be recommended, the matching human body model for the physiological feature information from the plurality of comparable human body models comprises:
determining the matching human body model from the plurality of comparable human body models based on the physiological feature information and living habit information of the user to be recommended.

**9.** The recommendation method according to claim 8, wherein the determining the matching human body model from the plurality of comparable human body models based on the physiological feature information and the living habit information of the user to be recommended comprises:

determining a plurality of candidate human body models from the plurality of comparable human body models based on the physiological feature information of the user to be recommended; and determining the matching human body model from the plurality of candidate human body models based on the living habit information of the user to be recommended;
or
determining a plurality of candidate human body models from the plurality of comparable human body models based on the living habit information of the user to be recommended; and determining the matching human body model from the plurality of candidate human body models based on the physiological feature information of the user to be recommended.

**10.** The recommendation method according to claim 8, wherein the determining the matching human body model from the plurality of comparable human body models based on the physiological feature information and the living habit information of the user to be recommended comprises:
determining the matching human body model from the plurality of comparable human body models, based on health condition information of the user to be recommended.

**11.** The recommendation method according to claim 10, wherein the determining the matching human body model from the plurality of comparable human body models based on the health condition information of the user to be recommended comprises:

determining a plurality of candidate human body models from the plurality of comparable human body models based on the physiological feature information of the user to be recommended; and determining the matching human body model from the plurality of candidate human body models based on the living habit information and the health condition information of the user to be recommended;

or

determining a plurality of candidate human body models from the plurality of comparable human body models based on the living habit information and the health condition information of the user to be recommended; and determining the matching human body model from the plurality of candidate human body models based on the physiological feature information of the user to be recommended.

12. The recommendation method according to any one of claims 1 to 10, further comprising:

obtaining physiological feature information after training of the user to be recommended after training for a preset period of time based on the fitness regimen information;

generating a human body model after training based on the physiological feature information after training;

determining a matching human body model for the human body model after training from the plurality of comparable human body models based on the human body model after training; and

determining new fitness regimen information for the user to be recommended based on fitness regimen information associated with the matching human body model for the human body model after training.

13. The recommendation method according to any one of claims 1 to 10, wherein the determining, based on the physiological feature information of the user to be recommended, the matching human body model for the physiological feature information from the plurality of comparable human body models comprises:

generating a user feature vector based on the physiological feature information of the user to be recommended;

determining a matching user from the plurality of comparable users based on a degree of similarity between the user feature vector and comparable feature vectors of the plurality of comparable users, the comparable feature vectors being generated based on physiological feature information of the comparable users; and

determining a comparable human body model of the matching user as the matching human body model.

14. The recommendation method according to claim 13, wherein the generating the user feature vector based on the physiological feature information of the user to be recommended comprises:

generating the user feature vector based on at least one of living habit information of the user to be recommended and a weight of the living habit information, or health condition information of the user to be recommended and a weight of the health condition information, and the physiological feature information of the user to be recommended and a weight of the physiological feature information, the comparable feature vector of the comparable user being generated based on at least one of living habit information of the comparable user and a weight of the living habit information, or health condition information of the comparable user and a weight of the health condition information, and physiological feature information of the comparable user and a weight of the physiological feature information.

15. The recommendation method according to claim 14, wherein

the weight of the health condition information of the user to be recommended is greater than the weight of the living habit information of the user to be recommended, and the weight of the living habit information of the user to be recommended is greater than the weight of the physiological feature information of the user to be recommended; and

the weight of the health condition information of the comparable user is greater than the weight of the living habit information of the comparable user, and the weight of living habit information of the comparable user is greater than the weight of the physiological feature information of the comparable user.

16. The recommendation method according to claim 13, wherein the determining the matching user from the plurality of comparable users based on the degree of similarity between the user feature vector and comparable feature vectors of the plurality of comparable users comprises:

determining, using a machine learning model, a user training target vector of the user to be recommended based on the user feature vector, the user training target vector comprising training target information corresponding to a body part which the user to be recommended wants to achieve;

determining a user feature matrix of the user to be recommended based on the user feature vector and the user training target vector; and

determining a matching user from the plurality of comparable users based on a degree of similarity between the user feature matrix and comparable feature matrices of the plurality of comparable users, the comparable feature matrices being generated based on comparable feature vectors and comparable training target vectors of the plurality of comparable users.

17. The recommendation method according to claim 16, wherein the machine learning model is trained using the comparable feature vectors as inputs and the comparable training target vectors as outputs.

18. The recommendation method according to any one of claims 1 to 10, further comprising:

determining a preference matrix based on degrees of preference of a comparable user for different fitness effect information; and

determining a match matrix based on degrees of match between fitness effect information and different fitness regimen information;

wherein the determining fitness regimen information for the user to be recommended based on fitness regimen information of the comparable user associated with the matching human body model comprises:

determining degrees of recommendation of the comparable user for different fitness regimen information based on the preference matrix and the match matrix; and

determining the fitness regimen information of the user to be recommended based on a degree of recommendation of a comparable user associated with the matching human body model selected by the user to be recommended.

19. A recommendation apparatus for fitness regimen information, comprising:

a match unit for, based on physiological feature information of a user to be recommended, determining a matching human body model for the physiological feature information from a plurality of comparable human body models, the plurality of comparable human body models being generated based on physiological feature information of a plurality of comparable users; and

a recommendation unit for determining fitness regimen information for the user to be recommended based on fitness regimen information of a comparable user associated with the matching human body model.

20. A recommendation apparatus for fitness regimen information, comprising:

a memory; and

a processor coupled to the memory, the processor configured to, based on instructions stored in the memory, carry out a recommendation method for fitness regimen information according to any one of claims 1 to 18.

21. A non-transitory computer-readable storage medium stored thereon a computer program that, when executed by a processor, implements a recommendation method for fitness regimen information according to any one of claims 1 to 18.

110

determine a matching human body
model

120

determine fitness regimen
information

**FIG. 1**

1110

determine a plurality of candidate
human body models

1120

determine a matching human body
model

**FIG. 2**

110

determine a matching human body model

120

determine fitness regimen information

330

obtain physiological feature information after training

FIG. 3

4

match unit 41

recommendation unit 42

FIG. 4

**FIG. 5**

**FIG. 6**

<div style="text-align:center">

**INTERNATIONAL SEARCH REPORT**

</div>

| International application No. |
|---|
| **PCT/CN2022/130641** |

| A. | CLASSIFICATION OF SUBJECT MATTER |
|---|---|

G16H 20/30(2018.01)i

According to International Patent Classification (IPC) or to both national classification and IPC

| B. | FIELDS SEARCHED |
|---|---|

Minimum documentation searched (classification system followed by classification symbols)

G16H; G06K; G06N

Documentation searched other than minimum documentation to the extent that such documents are included in the fields searched

Electronic data base consulted during the international search (name of data base and, where practicable, search terms used)

CNABS; CNTXT; VEN; USTXT; EPTXT; WOTXT; CNKI: 健身, 锻炼, 人体, 模型, 匹配, 类比, 方案, 建议, fitness, exercise, body, model, match, scheme, suggest

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 113782149 A (BEIJING BOE TECHNOLOGY DEVELOPMENT CO., LTD. et al.) 10 December 2021 (2021-12-10) description, paragraphs 52-149 | 1-21 |
| X | CN 108766524 A (SHENZHEN LINGDU INTELLIGENT CONTROL TECHNOLOGY CO., LTD.) 06 November 2018 (2018-11-06) description, paragraphs 48-152 | 1-21 |
| X | CN 105678064 A (BEIJING XIAOMI TECHNOLOGY CO., LTD.) 15 June 2016 (2016-06-15) description, paragraphs 66-143 | 1-21 |
| A | CN 112967783 A (TERMINUS GROUP CO., LTD.) 15 June 2021 (2021-06-15) entire document | 1-21 |
| A | US 2021343412 A1 (NIPPON TELEGRAPH AND TELEPHONE CORPORATION) 04 November 2021 (2021-11-04) entire document | 1-21 |

☐ Further documents are listed in the continuation of Box C.　　☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 January 2023** | **16 January 2023** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT
Information on patent family members

| International application No. |
| --- |
| **PCT/CN2022/130641** |

| Patent document cited in search report | | | Publication date (day/month/year) | Patent family member(s) | | | Publication date (day/month/year) |
| --- | --- | --- | --- | --- | --- | --- | --- |
| CN | 113782149 | A | 10 December 2021 | None | | | |
| CN | 108766524 | A | 06 November 2018 | None | | | |
| CN | 105678064 | A | 15 June 2016 | CN | 105678064 | B | 09 July 2019 |
| CN | 112967783 | A | 15 June 2021 | None | | | |
| US | 2021343412 | A1 | 04 November 2021 | WO | 2020044824 | A1 | 05 March 2020 |
| | | | | JP | 2020035365 | A | 05 March 2020 |
| | | | | JP | 7139795 | B2 | 21 September 2022 |

Form PCT/ISA/210 (patent family annex) (January 2015)

## REFERENCES CITED IN THE DESCRIPTION

**Patent documents cited in the description**

• CN 202111336086